# EUROPEAN PATENT APPLICATION

(11) **EP 4 239 335 A1**
(43) Date of publication of application: **06.09.2023**
(21) Application number: 21900614.5
(22) Date of filing: 30.11.2021
(51) Int. Cl.: G01N 33/536, C07K 16/18

(54) **BIOMOLECULE STRUCTURE DETECTION PROBE, BIOMOLECULE STRUCTURE DETECTION KIT, AND METHOD FOR DETECTING BIOMOLECULE STRUCTURE**

(30) Priority: 01.12.2020 JP 2020199800
(71) Applicant: Kyushu University, National University Corporation, Nishi-ku Fukuoka-shi Fukuoka 819-0395 (JP)
(72) Inventor: OHKAWA Yasuyuki, Fukuoka-shi, Fukuoka 819-0395 (JP); TOMIMATSU Kosuke, Fukuoka-shi, Fukuoka 819-0395 (JP)
(74) Representative: EP&C
(86) International application number: PCT/JP2021/043986
(87) International publication number: WO 2022/118862

(57) **Abstract**

A biomolecule structure detection probe, wherein a specific binding substance having a specific binding activity to a biomolecule structure is linked to a labeling substance via a linker including a disulfide bond. In addition, a biomolecule structure detection kit including the biomolecule detection probe and a reagent for cleaving the disulfide bond. In addition, a biomolecule structure detection kit including a linker for linking a specific binding substance having a specific binding activity to a biomolecule structure with a labeling substance, the linker including a disulfide bond; and a labeling substance capable of being bonded to the linker; and a reagent for cleaving the disulfide bond. In addition, a method for detecting a biomolecule structure using the biomolecule structure detection probe.

## Description

### [Technical Field]

The present invention relates to a biomolecule structure detection probe, a biomolecule structure detection kit, and a method for detecting a biomolecule structure.

Priority is claimed on Japanese Patent Application No. 2020-199800 filed December 1, 2020 in Japan, the content of which is incorporated herein by reference.

### [Background Art]

Fluorescent immunostaining method is a technique for detecting a state of expression of antigens in tissue samples. In the related art, in a case of detecting a plurality of types of antigens in the same sample, a plurality of types of fluorescent markers having mutually different maximum fluorescence wavelengths have been used. However, the types of fluorescent markers that can be used are limited. Even in a case where fluorescent markers having different maximum fluorescence wavelengths are used, there is a case where fluorescence that is not a target of detection leaks into a wavelength range of the fluorescence that is the target of detection, and accurate analysis of the state of expression of an antigen that is the target of detection is prohibited.

Patent Document 1 reports an antibody marked with a photo cleavable label. Patent Document 1 discloses a method of performing immunostaining with an antibody labeled with a photo cleavable label, detecting the photo cleavable label, and then performing irradiation with ultraviolet rays to separate the label.

### [Citation List]

### [Patent Document]

[Patent Document 1]
Published Japanese Translation No. 2018-523826 of the PCT International Publication

### [Summary of Invention]

### [Technical Problem]

In Patent Document 1, a photo cleavable label is used and the label is separated by irradiation with ultraviolet rays. However, in a case where a photo cleavable label is applied to fluorescent multiple staining, there is a risk that a photo cleavage site is cleaved by excitation light for detecting the fluorescent label, and the fluorescent label is unintentionally separated.

Accordingly, an object of the present invention is to provide a biomolecule structure detection probe, a biomolecule structure detection kit, and a method for detecting a biomolecule structure, in which the same labeling substance can be used repeatedly without using a photo cleavable label.

### [Solution to Problem]

The present invention includes the following aspects.
[1] A biomolecule structure detection probe, in which a specific binding substance having a specific binding activity to a biomolecule structure is linked to a labeling substance via a linker including a disulfide bond.
[2] The biomolecule structure detection probe according to [1], in which the specific binding substance is an antibody.
[3] The biomolecule structure detection probe according to [1] or [2], in which the labeling substance is a fluorescent dye.
[4] A biomolecule structure detection kit including: the biomolecule structure detection probe according to any one of [1] to [3]; and a reagent for cleaving a disulfide bond.
[5] A biomolecule structure detection kit including: a linker for linking a specific binding substance having a specific binding activity to a biomolecule structure with a labeling substance, the linker including a disulfide bond; a labeling substance bonded to or capable of being bonded to the linker; and a reagent for cleaving the disulfide bond.
[6] The biomolecule structure detection kit according to [5], in which the specific binding substance is an antibody.
[7] The biomolecule structure detection kit according to [5] or [6], in which the labeling substance is a fluorescent dye.
[8] A method for detecting a biomolecule structure including: step (A) of detecting a first biomolecule structure in a sample containing cells, using a first biomolecule structure detection probe in which a specific binding substance having a specific binding activity to the first biomolecule structure is linked to a first labeling substance via a linker including a disulfide bond; and step (B) of cleaving the disulfide bond in the first biomolecule structure detection probe to release the first labeling substance.
[9] The method for detecting a biomolecule structure according to [8], further including: step (C) of detecting a second biomolecule structure in the sample, using a second biomolecule structure detection probe in which the specific binding substance having a specific binding activity to the second biomolecule structure is linked to a second labeling substance via a linker including a disulfide bond, after the step (B).
[10] The method for detecting a biomolecule structure according to [9], in which the first labeling substance and the second labeling substance are the same labeling substances.
[11] The method for detecting a biomolecule structure according to any one of [8] to [10], in which the first biomolecule structure is a biomolecule structure included in a first primary probe that is specifically bound to a third biomolecule structure included in the cell.
[12] The method for detecting a biomolecule structure according to [9] or [10], in which the first biomolecule structure is a biomolecule structure included in a first primary probe that is specifically bound to a third biomolecule structure included in the cell, and the second biomolecule structure is a biomolecule structure included in a second primary probe that is specifically bound to a fourth biomolecule structure included in the cell.

### [Advantageous Effects of Invention]

According to the present invention, there is provided a biomolecule structure detection probe, a biomolecule structure detection kit, and a method for detecting a biomolecule structure, in which the same labeling substance can be repeatedly used without using a photo cleavable label.

### [Brief Description of Drawings]

FIG. 1 is a diagram schematically representing a method for detecting a biomolecule structure according to one embodiment.
FIG. 2 is a diagram schematically showing a method for detecting a biomolecule structure according to one embodiment.
FIG. 3 is a diagram schematically shows an outline of immunostaining method of Example 1.
FIG. 4 is a fluorescence image showing results of immunostaining of Example 1.
FIG. 5 is a fluorescence image showing results of immunostaining of Example 2.
FIG. 6 schematically shows an outline of immunostaining method of Example 3 and Comparative Example 1.
FIG. 7 is a fluorescence image showing results of immunostaining of Example 3.
FIG. 8 is a fluorescence image showing results of immunostaining of Comparative Example 1.
FIG. 9 is a fluorescence image showing results of immunostaining of Reference Example 1.
FIG. 10 is a fluorescence image showing results of serial immunostaining of Example 4.
FIG. 11 is proteome data obtained by quantifying, at a single-cell level, a signal of each fluorescent image obtained by performing serial immunostaining using about 60 types of biomolecule structure detection probes.
FIG. 12 shows results of dimensionality reduction (UMAP) of the data in FIG. 11 and classification of cells into five groups.
FIG. 13 is a diagram showing positions of cells present in fluorescent images of serial immunostaining as dots, and showing positions of cells with dots of different colors corresponding to each group.
FIG. 14 shows results of superimposing a quantitative value of the expression of a specific protein on cell position information obtained in FIG. 13.

### [Description of Embodiments]

Hereinafter, embodiments of the present invention will be described in detail with reference to the drawings depending on the case. In the drawings, the same or corresponding parts will be denoted by the same or corresponding reference numerals, and overlapping descriptions will be omitted. Dimensional ratios in each drawing are exaggerated for explanation and do not necessarily match the actual dimensional ratios.

The term "comprise" means that a constitutional element other than the constitutional element to be targeted may be encompassed. The term "consist of" means that a constitutional element other than the constitutional element to be targeted is not encompassed. The term "consist essentially of" means that a constitutional element other than the constitutional element to be targeted is not included as an aspect (aspect in which the effect of the invention is lost and the like) that exhibits a special function. In a case where "comprise" is described in the present specification, the aspect of "consist of" and the aspect of "consist essentially of" are included.

Proteins, peptides, nucleic acids, cells, and the like can be isolated. The term "isolated" means a natural state or a state of being separated from other components. "Isolated" one does not substantially include other components. The term "does not substantially include other components" means that a content of other components included in the isolated component is negligible. The content of other components included in the isolated component is, for example, 10% by mass or less, 5% by mass or less, 4% by mass or less, 3% by mass or less, 2% by mass or less, 1% by mass or less, 0.5% by mass or less, or 0.1 % by mass or less. The proteins, peptides, nucleic acids, and cells described in the present specification can be isolated proteins, isolated peptides, isolated nucleic acids, and isolated cells.

### [Biomolecule structure detection probe]

A first aspect of the present invention is a biomolecule structure detection probe, in which a specific binding substance having a specific binding activity to a biomolecule structure is linked to a labeling substance via a linker including a disulfide bond.

"Probe" means a molecule or molecule complex that is used to detect a specific biomolecule structure. The biomolecule structure detection probe of the present embodiment is a structure in which a specific binding substance having a specific binding activity to a biomolecule structure which is a target of detection is linked to a labeling substance via a linker including a disulfide bond.

The term "biomolecule" means an organic compound included in a living organism. A biomolecule may be a molecule that functions in a biological phenomenon. A biomolecule may be an artificially synthesized molecule that mimics a natural biomolecule. Examples of the biomolecule include peptides, proteins, nucleic acids, lipids, sugars, glycolipids, vitamins, hormones, amino acids, nucleotides, and the like.

The term "biomolecule structure" means a structure included in a biomolecule. The biomolecule structure may be a partial structure of a biomolecule, may be a partial structure of a primary structure, may be a partial structure of a secondary structure, or may be a partial structure of a tertiary structure. The biomolecule structure may be a partial structure of a three-dimensional structure composed of a plurality of biomolecules. In a case where the biomolecule is a peptide or protein, the biomolecule structure may be, for example, a partial region including a partial amino acid sequence of the protein, or may be a partial structure of a three-dimensional structure of the protein. In a case where some of amino acid residues in the protein are modified by phosphorylation, glycosylation, ubiquitination, methylation, acetylation, and the like, the amino acid residues may be a structure of the modified amino acid residues. In addition, in a case where the biomolecule is a nucleic acid, the biomolecule structure may be, for example, a partial nucleotide sequence of the nucleic acid.

The term "specific binding substance" means a substance that has a specific binding activity to a specific biomolecule structure. The term "having a specific binding activity" means having high binding affinity to a specific biomolecule structure, but has very low binding affinity to other biomolecule structures. A specific binding substance preferably has a high binding activity to a specific biomolecule structure, but has little binding activity to other biomolecule structures.

Examples of combinations of a biomolecule structure and a specific binding substance include a combination of a partial structure of peptide or protein and an antibody, an antibody fragment, or an antibody mimic; a combination of a partial sequence region of a nucleic acid and a nucleic acid including a sequence complementary to the partial sequence; a combination of a ligand and a receptor thereof; a combination of an enzyme and a base, an inhibitor, or a cofactor; a combination of a sugar chain and a lectin; a combination of a peptide, a protein, or a nucleic acid and an aptamer; a combination of a transcription control sequence portion of a nucleic acid and a transcription control factor thereof; and the like, but are not limited thereto.

An antibody may be of any class and subclass of immunoglobulin. Biological species from which the antibody is derived is not particularly limited, and the antibody may be derived from any living organism. The antibody is preferably a monoclonal antibody. The antibody may be a modified antibody such as a chimeric antibody.

An antibody fragment means an antibody fragment that retains an antigen-binding activity. Examples of the antibody fragment include scFv, Fab, F(ab')2, Fv, and the like, but are not limited thereto.

An antibody mimic refers to a non-immunoglobulin molecule that has a specific binding activity to an antigen, similar to an antibody. Examples of the antibody mimic include affibody molecule, affilin, affimer, affitin, alphabody, anticalin, avimer, DARPin, fynomer, Kunitz domain peptide, monobody, and the like, but are not limited thereto.

An aptamer is a substance having a specific binding activity to a target substance. Examples of the aptamer include a nucleic acid aptamer, a peptide aptamer, and the like. The nucleic acid aptamer can be selected by, for example, a systematic evolution of ligand by exponential enrichment (SELEX) method and the like. The peptide aptamer can be selected by, for example, the two-hybrid method using yeast.

As a specific binding substance, a substance having a specific binding activity to any biomolecule structure in cells can be used. For example, in a case where it is desired to detect a partial amino acid sequence region, a modified amino acid residue, or a partial three-dimensional structure included in a specific peptide or protein as a biomolecule structure, an antibody, an antibody fragment, an antibody mimic, an aptamer, or the like having a specific binding activity to these can be used as a specific binding substance. In a case where it is desired to detect a partial nucleotide sequence region of a specific mRNA or genomic DNA as a biomolecule structure, a nucleic acid including a nucleotide sequence complementary to the partial nucleotide sequence, an aptamer, or the like can be used as a specific binding substance.

A specific binding substance may have a specific binding activity to a primary probe. The term "primary probe" is a probe that is first bound to a biomolecule structure that is a target of detection. As the primary probe, biopolymers such as antibody, antibody fragment, and nucleic acid can be used, for example. In a case where the primary probe is an antibody (primary antibody), as the specific binding substance, antibodies (antibody, antibody fragment, antibody mimic, aptamer, and the like) having a specific binding activity to a constant region of the primary antibody can be used, for example.

The term "labeling substance" means a substance that directly or indirectly produces a detectable signal by chemical or physical means. Examples of the labeling substance include enzyme label such as peroxidase (for example, horseradish peroxidase) and alkaline phosphatase; fluorescent label such as carboxy fluorescein (FAM), 6-carboxy-4',5'-dichloro2',7'-dimethoxy fluorescein (JOE), fluorescein isothiocyanate (FITC), tetrachlorofluorescein (TET), 5'-hexachloro-fluorescein-CE phosphoramidite (HEX), Cy3, Cy5, Alexa 488, Alexa 555, Alexa 568, and Alexa 647; radioisotope label such as Iodine 125; electrochemiluminescent label such as ruthenium complex; metal nanoparticles; and the like, but are not limited thereto. Preferable examples of the labeling substance include fluorescent label (fluorescent dye).

The term "linker" means a linking moiety that links two substances or a molecule that is used to link two substances. In the biomolecule structure detection probe of the present embodiment, a specific binding substance is linked to a labeling substance via a linker including a disulfide bond.

The biomolecule structure detection probe of the present embodiment can be represented by Formula (P1), for example.

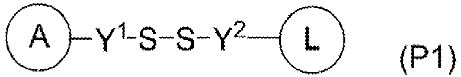

[In the formula, Y¹ and Y² each independently represent a divalent linking group; L represents a labeling substance; and A represents a specific binding substance.]

In Formula (Pl), Y¹ and Y² each independently represent a divalent linking group. The divalent linking group preferably includes a bond structure. The bond structure means a structure formed by bonding two functional groups by a chemical reaction or intermolecular interaction. Examples of the chemical reaction forming a bond structure include a dewatering condensation reaction, a cycloaddition reaction, and the like, but are not limited thereto. It is preferable that the bond structure including Y¹ and Y² does not include a disulfide bond. Examples of the bond structure include an amide bond (-CO-NH-), an ester bond (-CO-O-), a thioester bond (-CO-S-), a phosphate ester bond (-PO₂-O-), a urethane bond (-NH-CO-O-), a bond including a 1,2,3-triazole ring, and the like, but are not limited thereto. The bond structure may be a bond through an intermolecular interaction such as avidin-biotin bond.

The biomolecule structure detection probe of the present embodiment may be represented by Formula (P1-1), for example.

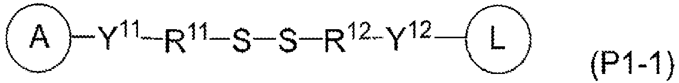

[In the formula, Y¹¹ and Y¹² each independently represent a divalent linking group including a bond structure; R¹¹ and R¹² each independently represent a divalent linking group; L represents a labeling substance; and A represents a specific binding substance.]

In Formula (P1-1), Y¹¹ and Y¹² each independently represent a divalent linking group including a bond structure. Examples of the bond structure include the same ones as those mentioned in Y¹ and Y² described above.

In Formula (P1-1), R¹¹ and R¹² each independently represent a divalent linking group. Examples of the divalent linking group include a hydrocarbon group which may have a substituent. The hydrocarbon group may be an aliphatic hydrocarbon group, or may be an aromatic hydrocarbon group.

The aliphatic hydrocarbon group may be saturated, or may be unsaturated, but is preferably saturated. Examples of the aliphatic hydrocarbon group include a linear or branched aliphatic hydrocarbon group, an aliphatic hydrocarbon group including a ring in the structure, and the like.

The number of carbon atoms of the linear aliphatic hydrocarbon group is preferably 1 to 15, more preferably 1 to 10, even more preferably 1 to 6, and particularly preferably 1 to 3. The linear aliphatic hydrocarbon group is preferably a linear alkylene group.

The number of carbon atoms of the branched aliphatic hydrocarbon group is preferably 2 to 15, more preferably 2 to 10, and further more preferably 3 to 6. The branched aliphatic hydrocarbon group is preferably a branched alkylene group.

Examples of the aliphatic hydrocarbon group including a ring in the structure include a cyclic aliphatic hydrocarbon group which may include a substituent including a heteroatom in the ring structure (a group obtained by removing two hydrogen atoms from an aliphatic hydrocarbon ring), a group obtained by bonding the cyclic aliphatic hydrocarbon group to a terminal of a linear or branched aliphatic hydrocarbon group, a group obtained by interposing the cyclic aliphatic hydrocarbon group in a linear or branched aliphatic hydrocarbon group, and the like. Examples of the linear or branched aliphatic hydrocarbon group include the same ones as those described above. The number of carbon atoms of the cyclic aliphatic hydrocarbon group is preferably 3 to 20, and more preferably 3 to 12. The cyclic aliphatic hydrocarbon group may be a polycyclic group, or may be a monocyclic group. In the cyclic aliphatic hydrocarbon group, some of the carbon atoms constituting the ring structure thereof may be substituted with a substituent including a heteroatom (oxygen atom, nitrogen atom, sulfur atom, and the like).

In a case where the divalent linking group for R¹¹ and R¹² is an aromatic hydrocarbon group, the number of carbon atoms is further more preferably 6 to 15, and particularly preferably 6 to 12. The aromatic hydrocarbon group is a hydrocarbon group including an aromatic ring. Examples of the aromatic ring include aromatic hydrocarbon rings such as benzene, naphthalene, anthracene, and phenanthrene; and aromatic heterocycles such as triazole ring, pyridine ring, and thiophene ring.

Specific examples of the aromatic hydrocarbon group include a group (arylene group or heteroarylene group) obtained by removing two hydrogen atoms from the aromatic hydrocarbon ring or aromatic heterocyclic ring described above; a group obtained by removing two hydrogen atoms from an aromatic compound including two or more aromatic rings (for example, biphenyl, fluorene, and the like); a group (group obtained by further removing one hydrogen atom from an aryl group or heteroaryl group) substituting one of hydrogen atoms of a group (aryl group or heteroaryl group) obtained by removing one hydrogen atom from the aromatic hydrocarbon ring or aromatic heterocyclic ring with an alkylene group, and the like. The number of carbon atoms of the alkylene group substituting the hydrogen atom is preferably 1 to 10, more preferably 1 to 6, and further more preferably 1 to 4.

In the hydrocarbon group which may have a substituent, some of the hydrogen atoms in the hydrocarbon chain may be substituted with a monovalent group, and some of a methylene group (-CH₂-) constituting the hydrocarbon chain may be substituted with a divalent group including a heteroatom. Examples of monovalent groups that substitute hydrogen atoms include acyl group, alkoxy group, hydroxy group, carboxy group, amino group, thiol group, and the like, but are not limited thereto. Examples of the divalent group substituting the methylene group include -O-, -C(=O)-O-, -O-C(=O)-, -C(=O)-, -O-C(=O)-O-, -C(=O)-NH-, -NH-C(=O)-, -NH-, and the like.

Specific examples of the biomolecule structure detection probe of the present embodiment include those represented by Formula (P1-1-1), but are not limited thereto.

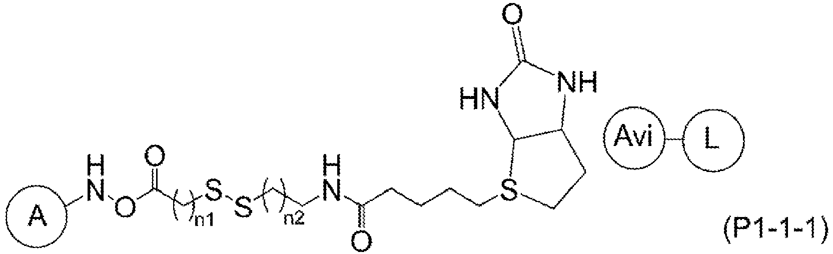

[In the formula, n1 and n2 each independently represent an integer of 1 to 10; Avi represents avidin or a derivative thereof; L represents a labeling substance; and A represents a specific binding substance.]

In Formula (P1-1-1), Avi represents avidin or a derivative thereof. Examples of the avidin derivative include streptavidin, neutravidin, and the like. Avidin or a derivative thereof represented by Avi is bonded to the biotin moiety through intermolecular interaction.

In Formula (P1-1-1), n1 and n2 are each independently an integer of 1 to 10. n1 and n2 are preferably integers of 1 to 6, more preferably integers of 1 to 3, and even more preferably integers of 2 or 3.

Specific examples of the biomolecule structure detection probe of the present embodiment are mentioned below, but are not limited thereto.

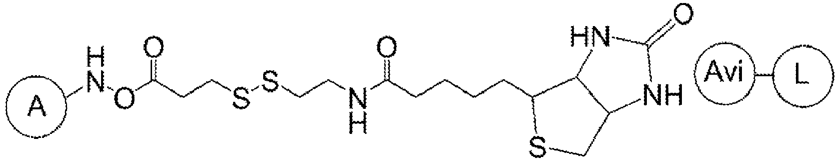

[In the formula, Avi represents avidin or a derivative thereof; L represents a labeling substance; and A represents a specific binding substance.]

Since the biomolecule structure detection probe of the present embodiment includes a disulfide bond in the linker moiety between the specific binding substance and the labeling substance, it is possible to separate the labeling substance at any timing by cleaving the disulfide bond. The disulfide bond can be easily cleaved with a reducing agent such as tris(2-carboxyethyl)phosphine (TCEP), 2-mercaptoethanol, dithiothreitol (DTT), and the like. Since the disulfide bond is not cleaved by light, cleavage is not occurred in a case of performing irradiation with excitation light required at the time of performing fluorescence observation. Since ultraviolet rays are not used to cleave the disulfide bond, biomolecules such as nucleic acid are not denatured by ultraviolet rays. Therefore, after detecting a specific living organism structure using the biomolecule structure detection probe of the present aspect, cells can be collected by a microdissection method and the like, and transcriptome analysis and the like can be suitably performed.

### [Biomolecule structure detection kit]

### <First embodiment>

A second aspect of the present invention is a biomolecule structure detection kit including a biomolecule detection probe of the above aspect and a reagent for cleaving a disulfide bond.

### (Biomolecule detection probe)

The biomolecule detection probe is the same as the biomolecule detection probe of the above aspect. In the biomolecule detection probe included in the kit of the present embodiment, a specific binding substance may be, for example, a specific binding substance having a specific binding activity (antibody, antibody fragment, antibody mimic, aptamer, or the like) to a primary probe (for example, primary antibody). For example, in a case where the primary probe is an antibody derived from a specific species of animal (for example, mouse antibody), as the specific binding substance of the biomolecule detection probe, an antibody (for example, goat anti-mouse antibody) derived from other species of animals having a specific binding activity to a constant region of the antibody, an antibody fragment thereof, or the like can be used.

### (Reagent for cleaving)

The biomolecule structure detection kit of the present embodiment includes a reagent for cleaving a disulfide bond in addition to the biomolecule detection probe described above. The reagent for cleaving a disulfide bond is not particularly limited as long as it is capable of cleaving a disulfide bond. Examples of the reagent for cleaving include a reducing agent such as TCEP, 2-mercaptoethanol, DTT, and the like. As the reagent for cleaving, TCEP is preferable since TCEP has high stability and selectivity.

### <Second embodiment>

A third aspect of the present invention is a biomolecule structure detection kit including: a linker for linking a specific binding substance having a specific binding activity to a biomolecule structure with a labeling substance, the linker including a disulfide bond; a labeling substance bonded to or capable of being bonded to the linker; and a reagent for cleaving the disulfide bond.

### (Linker)

The kit of the present embodiment includes a linker for linking a specific binding substance having a specific binding activity to a biomolecule structure with a labeling substance. The linker may have, for example, a functional group that reacts with a functional group included in a specific binding substance or labeling substance. For example, in a case where the specific binding substance has an amino group, the linker may have an amine-reactive group. The amine-reactive group is a functional group that reacts with amine. The amine-reactive group is not particularly limited, and known amine-reactive groups can be used. Examples of the amine-reactive group include N-hydroxyester (NHS-ester) group, carboxy group, isocyanate group, isothiocyanate group, sulfonyl chloride group, aldehyde group, carbodiimide group, acyl azide group, epoxy group, imide ester group, and the like, but are not limited thereto.

Bonding between the linker and the specific binding substance or labeling substance may be performed by avidin-biotin bond. In this case, the linker may include a group derived from biotin.

Examples of the linker include those represented by the Formula (L1).

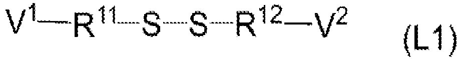

[In the formula, V¹ and V² each independently represent a functional group or a group derived from biotin; R¹¹ and R¹² each independently represent a divalent linking group.]

In the formula (L1), V¹ and V² each independently represent a functional group or a group derived from biotin. The functional group in V¹ and V² is a functional group capable of forming a bond structure by reacting with the functional group of the specific binding substance or labeling substance. For example, in a case where the specific binding substance or labeling substance has an amino group, examples of the functional group in V¹ and V² include an amine-reactive group as described above.

In Formula (L1), R¹¹ and R¹² each independently represent a divalent linking group. R¹¹ and R¹² are the same as R¹¹ and R¹² in Formula (P1-1) described above.

### (Labeling substance)

A labeling substance may be bonded to the linker. Alternatively, the labeling substance may be provided in a state of not being bonded to the linker. In this case, the labeling substance has a structure capable of being bonded to the linker. For example, the labeling substance may have a functional group capable of forming a bond structure by reacting with the functional group of the linker. Alternatively, in a case where the linker includes a group derived from biotin, avidin or an avidin derivative may be attached. Examples of the avidin derivative include the same as those mentioned above. In a case where the labeling substance is provided in a state of not being bonded to the linker, a user may perform a ligation between the linker and the labeling substance before use.

### (Reagent for cleaving)

As the reagent for cleaving, the same one as mentioned in the kit of the first embodiment can be used.

The kit of the present embodiment does not include a specific binding substance, and a user can select any specific substance. A user can prepare a biomolecule detection probe by performing a bonding reaction between any specific binding substance and a linker before use.

### (Optional element)

The kit according to the second aspect or the third aspect may include other elements in addition to the above elements. Examples of the other elements include a reagent for detection of a labeling substance, a reagent for preparation, a diluent, a buffer (blocking buffer, washing buffer, and the like), instructions for use, and the like.

The kit of the present embodiment can be used for a method for detecting a biomolecule structure described below.

### [Method for detecting biomolecule structure]

A fourth aspect of the present invention is a method for detecting a biomolecule structure, including step (A) of detecting a first biomolecule structure in a sample containing cells, using a first biomolecule structure detection probe, in which a specific binding substance having a specific binding activity to the first biomolecule structure is linked to a first labeling substance via a linker including a disulfide bond; and step (B) of cleaving the disulfide bond in the first biomolecule structure detection probe to release the first labeling substance.

The method of the present embodiment is a method for detecting a targeted biomolecule structure in a sample containing cells. The term "sample containing cells" is not particularly limited as long as the term is a sample containing cells. The sample containing cells may be a tissue section, may be a cell suspension, may be a body fluid sample containing cells, and the like. The cell may be a cell of any living organism.

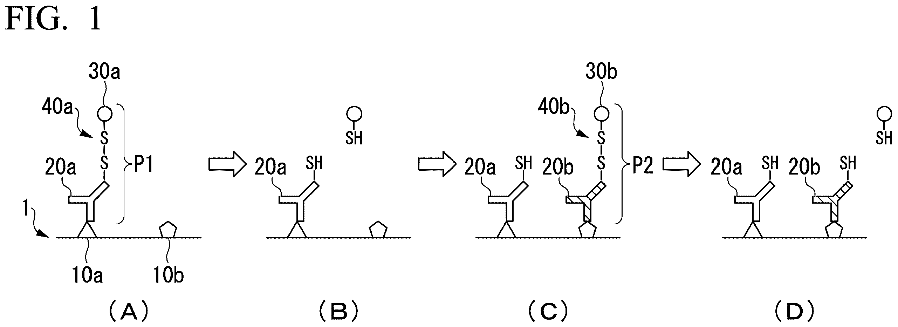

FIG. 1 is a diagram schematically showing an example of the method of the present embodiment. The method of the present embodiment can be carried out using the biomolecule structure detection probe of the first aspect. In FIG. 1, 1 is a sample containing cells. A sample 1 includes biomolecules 10a and 10b. The biomolecules 10a and 10b are biomolecules including biomolecule structures that are targets of detection, respectively. 20a is a specific binding substance that specifically binds to the biomolecule structure included in the biomolecule 10a. 30a is a labeling substance. The specific binding substance 20a is linked to the labeling substance 30a via a linker 40a including a disulfide bond to form a biomolecule structure detection probe P1. 20b is a specific binding substance that specifically binds to a biomolecule structure included in the biomolecule 10b. 30b is a labeling substance. The specific binding substance 20b is linked to the labeling substance 30b via a linker 40b including a disulfide bond to form a biomolecule structure detection probe P2.

Examples of the biomolecules 10a and 10b are the same as those exemplified in the [biomolecule structure detection probe] described above. The biomolecules 10a and 10b are, for example, peptides or proteins. Examples of the specific binding substances 20a and 20b are the same as those exemplified in the [biomolecule structure detection probe] described above. The specific binding substances 20a and 20b are, for example, antibodies or antibody fragments. Examples of the labeling substances 30a and 30b are the same as those exemplified in the [biomolecule structure detection probe] described above. The labeling substances 30a and 30b are, for example, fluorescent dyes. The linkers 40a and 40b are the same as those exemplified in the [biomolecule structure detection probe] described above. The labeling substances 30a and 30b may be the same or different. In a case where the labeling substance 30a is a fluorescent dye, the labeling substance 30b is also preferably a fluorescent dye. In this case, the fluorescent dyes of the labeling substances 30a and 30b may be the same or different.

### <Step (A)>

In step (A), a first biomolecule structure (biomolecule structure (biomolecule structure included in a biomolecule 10a) in a sample (sample 1) containing cells is detected using a first biomolecule structure detection probe (biomolecule structure detection probe P1) in which a specific binding substance (specific binding substance 20a) having a specific binding activity to a first biomolecule structure is linked to a first labeling substance (labeling substance 30a) via a linker (linker 40a) including a disulfide bond (refer to FIG. 1(A)).

In FIG. 1, the biomolecule structure detection probe P1 is the first biomolecule structure detection probe. In step (A), the sample 1 is treated with the biomolecule structure detection probe P1. With this, the biomolecule structure detection probe P1 is bound to the biomolecule 10a via the specific binding substance 20a.

A method of treating the sample 1 with the biomolecule structure detection probe P1 can be appropriately selected according to the type of the specific binding substance 20a. For example, a solution of the biomolecule structure detection probe P1 obtained by dissolving the biomolecule structure detection probe P1 in an appropriate buffer (for example, phosphate buffer, trishydrochloric acid buffer, PBS, and the like) is added to sample 1 and incubated. With this, the biomolecule structure detection probe P1 can be bound to the biomolecule 10a. The incubation temperature and incubation time can be appropriately selected according to the type of the specific binding substance 20a. For example, in a case where the specific binding substance 20a is an antibody or antibody fragment, the incubation temperature may be 20°C to 40°C (preferably 30°C to 40°C). The incubation time may be about 30 to 120 minutes.

After the treatment with the biomolecule structure detection probe P1, the sample 1 may be washed with a washing buffer and the like. With this, the unbound biomolecule structure detection probe P1 can be removed.

Before the treatment with the biomolecule structure detection probe P1, blocking of the sample 1 may be performed with a blocking agent. Non-specific bond of the biomolecule structure detection probe P1 can be reduced by performing blocking. Blocking agents include, but are not limited to, bovine serum albumin, skimmed milk, casein, gelatin, and the like.

Subsequently, the signal of the labeling substance 30a of the biomolecule structure detection probe P1 is detected. By detecting a signal of the labeling substance 30a, a biomolecule structure to which the specific binding substance 20a is bound can be indirectly detected. A method for detecting the signal of the labeling substance 30a can be appropriately selected according to the type of the labeling substance 30a. In a case where the labeling substance 30a is a fluorescent dye, the signal of the labeling substance 30a can be detected by performing irradiation with light having an excitation wavelength of the fluorescent dye and detecting fluorescence using a fluorescence microscope.

### <Step (B)>

In step (B), the disulfide bond in the first biomolecule structure detection probe (biomolecule structure detection probe P1) is cleaved to release the first labeling substance (labeling substance 30a) (refer to FIG. 1(B)).

Cleaving of the disulfide bond in the biomolecule structure detection probe P1 can be performed using a reagent for cleaving the disulfide bond. Examples of the reagent for cleaving include the same ones mentioned those the same one as mentioned in the [biomolecule structure detection kit]. The concentration of the reagent for cleaving is not particularly limited, and can be appropriately selected according to the type of the reagent for cleaving. The concentration of the reagent for cleaving may be an amount sufficient for cleaving the disulfide bond in the biomolecule structure detection probe P1. In a case where a reducing agent (TCEP, 2-mercaptomethanol, DTT, or the like) is used as the reagent for cleaving, the concentration of the reducing agent can be, for example, 5 mM or higher, 10 mM or higher, 20 mM or higher, or 30 mM or higher. The upper limit of the concentration of the reducing agent is not particularly limited, and can be, for example, 100 mM or less, 80 mM or less, 70 mM or less, 60 mM or less, or 50 mM or less. A treatment time with the reducing agent can be, for example, 10 minutes or longer, 15 minutes or longer, 20 minutes or longer, 25 minutes or longer, or 30 minutes or longer. The upper limit of the treatment time with the reducing agent is not particularly limited, and, from a viewpoint of not denaturing the biomolecules, can be, for example, 200 minutes or less, 150 minutes or less, or 120 minutes or less. In a case where the reducing agent is TCEP, for example, the concentration can be 5 to 50 mM and the treatment time can be about 20 to 40 minutes. In addition, examples of the treatment temperature include 20°C to 40°C.

By cleaving the disulfide bond in the biomolecule structure detection probe P1, the labeling substance 30a is separated from the biomolecule structure detection probe P1 and released. Therefore, the signal of the labeling substance 30a in the sample 1 disappears.

After treatment with the reagent for cleaving, the sample may be washed with a washing buffer and the like. With this, the released labeling substance 30a can be removed.

### <Optional steps>

The method of the present embodiment may include other steps in addition to the steps (A) and (B). Examples of other steps include step (C) of detecting a second biomolecule structure in the sample, using a second biomolecule structure detection probe in which a specific binding substance having a specific binding activity to a second biomolecule structure is linked to a second labeling substance via a linker including a disulfide bond (refer to FIG. 1(C)); step (D) of cleaving the disulfide bond in a biomolecule structure detection probe P2 to release the second labeling substance (refer to FIG. 1(D)); and the like.

### (Step (C))

The method of the present embodiment can further include the step (C) after the step (B). In FIG. 1, the biomolecule structure detection probe P2 is the second biomolecule structure detection probe. In the step (C), the sample 1 after the step (B) is treated with the biomolecule structure detection probe P2. With this, the biomolecule structure detection probe P2 is bound to the biomolecule 10b via the specific binding substance 20b.

The step (C) can be performed in the same manner as the step (A), except that the biomolecule structure detection probe P2 is used instead of the biomolecule structure detection probe P1.

After treating the sample 1 with the biomolecule structure detection probe P2, the signal of the labeling substance 30b of the biomolecule structure detection probe P2 is detected. By detecting the signal of the labeling substance 30b, the biomolecule structure to which the specific binding substance 20b is bound can be indirectly detected. A method for detecting the signal of the labeling substance 30b can be appropriately selected according to the type of the labeling substance 30b. In a case where the labeling substance 30b is a fluorescent dye, the signal of the labeling substance 30b can be detected by performing irradiation with light having an excitation wavelength of the fluorescent dye and detecting fluorescence using a fluorescence microscope.

The labeling substance 30b may be the same as or different from the labeling substance 30a. In the method of the present embodiment, the labeling substance 30a disappears from the sample 1 by the step (B). Therefore, even if the labeling substance 30b is the same as the labeling substance 30a, only the labeling substance 30b bound to the biomolecule 10b can be detected in the step (C).

### (Step (D))

The method of the present embodiment can further include the step (D) after the step (C). By performing the step (D), the signal of the labeling substance 30b in the sample 1 can disappear. The step (D) can be performed in the same manner as the step (B).

### (Repeated step)

The method of the present embodiment may further include step (E) of repeating the steps (C) and (D) by changing the type of the specific binding substance in the biomolecule structure detection probe. As the specific binding substance, a different specific binding substance is preferably used for each cycle of the steps (C) and (D). The labeling substance in the biomolecule structure detection probe may or may not be changed for each cycle. A number of repetitions of the steps (C) and (D) is not particularly limited, and can be any number of times. The number of repetitions of the steps (C) and (D) is, for example, 1 or more, 2 or more, 3 or more, 5 or more, 10 or more, 20 or more, 30 or more, 40 or more, or 50 or more. The upper limit of the number of repetitions of the steps (C) and (D) is not particularly limited, but may be, for example, 500 times or less, 400 times or less, 300 times or less, 200 times or less, or 100 times or less. The number of repetitions of the step (C) and (D) is, for example, 1 to 100 times, 1 to 90 times, 1 to 80 times, 1 to 70 times, 1 to 60 times, 1 to 50 times, 1 to 40 times, 1 to 30 times, 1 to 20 times, 1 to 10 times, 1 to 5 times, or the like.

In the method of the present embodiment, after detecting the first biomolecule structure using the first biomolecule structure detection probe, the disulfide bond in the first biomolecule structure detection probe is cleaved to release the labeling substance. Therefore, in a case where the second biomolecule structure is detected using the second biomolecule structure detection probe, the labeling substance of the first biomolecule structure detection probe does not interfere. Therefore, the same sample can be used to repeatedly perform detection operation of the biomolecule structure. In the method of the present embodiment, the same labeling substance can be used repeatedly, and thus the number of repetitions of the detection operation is not limited to the type of the labeling substance.

In the method of the present embodiment, since the disulfide bond is used for a structure for separating the labeling substance, even in a case where the fluorescent dye is used for the labeling substance, the labeling substance is not separated by excitation light. In the method of the present embodiment, separation of the labeling substance can be performed under reduction conditions of not denaturing the biomolecules. Therefore, after specifying a cell having a desired biomolecule structure by the method of the present embodiment, RNA and the like can be extracted from the cell and suitably used for transcriptome analysis and the like.

### <Modification example>

The method of the present embodiment can also be performed using a primary probe. In this case, the biomolecule structure to which the biomolecule structure detection probe is bound may be a biomolecule structure included in the primary probe. For example, the first biomolecule structure to which the first biomolecule structure detection probe is bound may be a biomolecule structure included in the first primary probe. In this case, the first primary probe may be specifically bound to a third biomolecule structure included in cells in the sample. The second biomolecule structure to which the second biomolecule structure detection probe is bound may be a biomolecule structure included in the second primary probe. In this case, the second primary probe may be specifically bound to a fourth biomolecule structure included in cells in the sample.

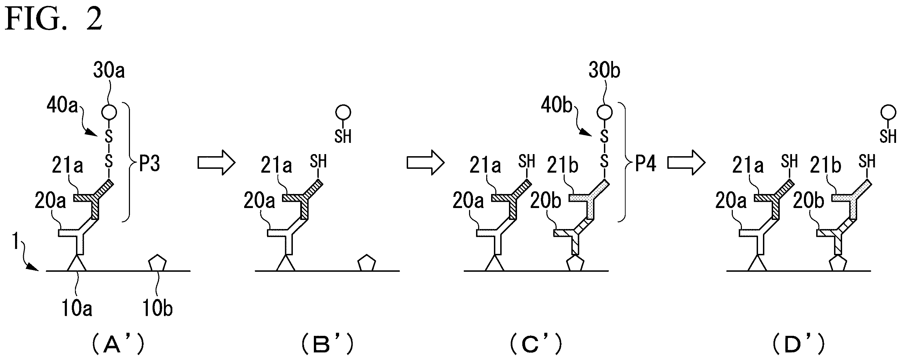

FIG. 2 is a diagram schematically showing an example of a method using a primary probe. In FIG. 2, the specific binding substance 20a is used as a first primary probe that is bound to the biomolecule structure included in the biomolecule 10a. 21a is a specific binding substance having specific bonding activity to the biomolecule structure of the specific binding substance 20a (first primary probe). The specific binding substance 21a is linked to the labeling substance 30a via a linker 40a including a disulfide bond to form a biomolecule structure detection probe P3. In the present modification example, the biomolecule structure detection probe P3 is used as the first biomolecule structure detection probe. The specific binding substances 20a and 21a are, for example, antibodies or antibody fragments.

In FIG. 2, the specific binding substance 20b is used as a second primary probe that is bound to the biomolecule structure included in the biomolecule 10b. 21b is a specific binding substance having specific binding activity to the biomolecule structure of the specific binding substance 20b (second primary probe). The specific binding substance 21b is linked to the labeling substance 30b via a linker 40b including a disulfide bond to form a biomolecule structure detection probe P4. In the present modification example, the biomolecule structure detection probe P4 is used as a second biomolecule structure detection probe. The specific binding substances 20b and 21b are, for example, antibodies or antibody fragments.

### (Step (A'): refer to FIG. 2(A'))

The method of the present modification example includes step (A'). Step (A') includes treating a sample (sample 1) containing cells using a first primary probe (specific binding substance 20a) having a specific binding activity to a third biomolecule structure (biomolecule structure included in biomolecule 10a) to bond the first primary probe to the third biomolecule structure in the sample; binding a first biomolecule structure detection probe (biomolecule structure detection probe P3), in which the specific binding substance (specific binding substance 21a) having a specific binding activity to the first biomolecule structure included in the first primary probe is linked to the first labeling substance (labeling substance 30a) via a linker (linker 40a) including a disulfide bond, to the first primary probe; and detecting the first biomolecule structure by detecting a signal of the first labeling substance.

In the step (A') of the present modification example, first, the sample 1 is treated with the specific binding substance 20a as a first primary probe. With this, the specific binding substance 20a is bound to the biomolecule 10a in the sample 1.

Subsequently, the sample 1 is treated with the biomolecule structure detection probe P3. With this, the biomolecule structure detection probe P3 is bound to the specific binding substance 20a via the specific binding substance 21a. As a result, a complex of the biomolecule 10a, the first primary probe (specific binding substance 20a), and the biomolecule structure detection probe P3 is formed.

A method of treating the sample 1 with the specific binding substance 20a and the biomolecule structure detection probe P3 can be performed in the same manner as the treatment of the sample 1 with the biomolecule structure detection probe P1 in the step (A).

After the treatment with the first primary probe (specific binding substance 20a), the sample 1 may be washed with a washing buffer and the like. With this, the unbound specific binding substance 20a can be removed. In addition, after the treatment with the biomolecule structure detection probe P3, the sample 1 may be washed with a washing buffer and the like. With this, the unbound biomolecule structure detection probe P3 can be removed.

Blocking of the sample 1 with a blocking agent may be performed before the treatment with the first primary probe (specific binding substance 20a). Non-specific bond of the specific binding substance 20a can be reduced by blocking. Blocking of the sample 1 may be performed with a blocking agent before the treatment with the biomolecule structure detection probe P3. Non-specific bond of the biomolecule structure detection probe P3 can be reduced by blocking. Examples of the blocking agent include the same as those as described above.

Subsequently, the signal of the labeling substance 30a of the biomolecule structure detection probe P3 is detected. By detecting the signal of the labeling substance 30a, the biomolecule structure of the biomolecule 10a bound via the specific binding substance 20a and the specific binding substance 21a can be indirectly detected. The method for detecting the signal of the labeling substance 30a can be performed in the same manner as in the step (A).

### (Step (B'): refer to FIG. 2(B'))

In step (B'), the disulfide bond in the first biomolecule detection structure probe (biomolecule structure detection probe P3) is cleaved to release the first labeling substance (labeling substance 30a). The step (B') can be performed in the same manner as the step (B).

### (Optional steps)

### <<Step (C'): refer to FIG. 2(C')>>

The method of the present modification example may include step (C') in addition to the steps (A') and (B'). The step (C') includes treating a sample (sample 1) containing cells using a second primary probe (specific binding substance 20b) having a specific binding activity to a fourth biomolecule structure (biomolecule structure included in biomolecule 10b) to be bound the second primary probe to the second biomolecule structure in the sample; binding a second biomolecule structure detection probe (biomolecule structure detection probe P4), in which the specific binding substance (specific binding substance 21b) having a specific binding activity to the second biomolecule structure included in the second primary probe is linked to the second labeling substance (labeling substance 30b) via a linker (linker 40b) including a disulfide bond, to the second primary probe; and detecting the second biomolecule structure by detecting a signal of the second labeling substance.

In the step (C') of the present modification example, the sample 1 after the step (B') is treated with the specific binding substance 20b as a second primary probe. With this, the specific binding substance 20b is bound to the biomolecule 10b in the sample 1.

Subsequently, the sample 1 is treated with the biomolecule structure detection probe P4. With this, the biomolecule structure detection probe P4 is bound to the specific binding substance 20b via the specific binding substance 21b. As a result, a complex of the biomolecule 10b, the second primary probe (specific binding substance 20b), and the biomolecule structure detection probe P4 is formed.

The method of treating the sample 1 with the specific binding substance 20b can be performed in the same manner as in the step (A'), except that the specific binding substance 20b is used instead of the specific binding substance 20a. The method of treating the sample 1 with the biomolecule structure detection probe P4 can be performed in the same manner as in the step (A'), except that the biomolecule structure detection probe P4 is used instead of the biomolecule structure detection probe P3.

Subsequently, the signal of the labeling substance 30b of the biomolecule structure detection probe P4 is detected. By detecting the signal of the labeling substance 30b, the biomolecule structure of the biomolecule 10b bound via the specific binding substance 20b and the specific binding substance 21b can be indirectly detected. The method for detecting the signal of the labeling substance 30b can be performed in the same manner as in the step (A).

### «Step (D'): refer to FIG. 2 (D')»

The method of the present modification example may include step (D') after the step (C'). In the step (D'), the disulfide bond in the second biomolecule structure detection probe (biomolecule structure detection probe P4) is cleaved to release the first labeling substance (labeling substance 30b). The step (D') can be performed in the same manner as the step (D).

### <<Repeating step>>

The method of the present embodiment may further include step (E') of changing the type of the primary probe and the type of the specific binding substance in the biomolecule structure detection probe to perform the step (C') and the step (D') repeatedly. It is preferable to use different specific binding substances in the primary probe and the biomolecule structure detection probe for each cycle of the step (C') and the step (D'). The labeling substance in the biomolecule structure detection probe may or may not be changed for each cycle. The number of repetitions of the step (C') and the step (D') is not particularly limited, and can be any number of times. The number of repetitions of the step (C') and the step (D') may be, for example, 1 time or more, 2 times or more, 3 times or more, 5 times or more, 10 times or more, 20 times or more, 30 times or more, 40 times or more, or 50 times or more. The upper limit of the number of repetitions of the step (C') and the step (D') is not particularly limited, but may be, for example, 500 times or less, 400 times or less, 300 times or less, 200 times or less, or 100 times or less. The number of repetitions of the step (C') and the step (D') is, for example, 1 to 100 times, 1 to 90 times, 1 to 80 times, 1 to 70 times, 1 to 60 times, 1 to 50 times, 1 to 40 times, 1 to 30 times, 1 to 20 times, 1 to 10 times, 1 to 5 times, or the like.

In the present modification example, the biomolecule structure detection probe is bound to the biomolecule structure in the sample which is a target of detection via the primary probe. Therefore, any biomolecule structure can be detected by using the primary probe having a specific binding activity to any biomolecule structure. For example, in a case where a substance including a specific biomolecule structure is used as the specific binding substance used for the primary probe, a substance including a specific binding substance to the specific biomolecule structure may be used as the biomolecule structure detection probe. For example, in a case where a mouse antibody is used as the primary probe, an antibody that binds to a constant region of the mouse antibody can be used as the specific binding substance included in the biomolecule structure detection probe. Therefore, as the biomolecule structure detection probe, one prepared in advance according to the type of the primary probe can be used.

### [Examples]

Hereinafter, the present invention will be described with reference to examples, but the present invention is not limited to the following examples.

### [Example 1]

### <Preparation of first biomolecule structure detection probe>

### (Preparation of linker)

As a linker, linker (1) having the following structure was used.

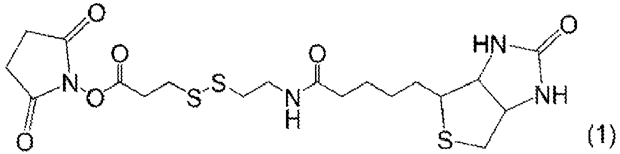

As the linker, a commercially available EZ-link Sulfo-NHS-SS-Biotin (Thermo Fisher) was used.

### (Bonding of linker and specific binding substance)

As the first specific binding substance, rat anti-mouse IgG antibody (Rat anti-mouse IgG, Jackson Immuno Research) was used. Bonding between the linker and the first specific binding substance was performed according to instructions attached to the linker.

### (Bonding of linker and labeling substance)

As a labeling substance, FITC-marked avidin (Avidin-FITC, Funakoshi) or Alexa 555-marked avidin (Streptavidin, Alexa Fluor 555 conjugate, Thermo Fisher) was used. The linker and the labeling substance were bonded by being reacted in a 0.1 M sodium hydrogen carbonate aqueous solution (pH 8.3) at room temperature for 30 minutes.

### <Preparation of second biomolecule structure detection probe>

The second biomolecule structure detection probe was prepared in the same method as the preparation method of the first biomolecule structure detection probe, except that a goat anti-rabbit IgG antibody (Goat anti-rabbit IgG, Jackson Immuno Research) was used as a specific binding substance.

### <Immuno staining>

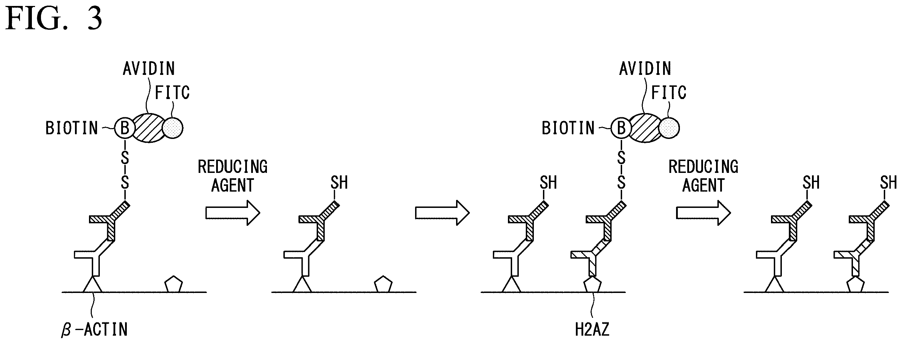

An outline of an immunostaining method of Example 1 is schematically shown in FIG. 3. A sample was reacted with a mouse anti-β-actin antibody (Anti-βActin, Abcam) as a primary antibody (first primary probe), and then reacted with the first biomolecule structure detection probe as a secondary antibody. After that, the sample was treated with 50 mM TCEP-HCl for 30 minutes. Subsequently, a rabbit anti-H2AZ antibody (Anti-H2AZ, Abeam) was reacted with the sample as a primary antibody (second primary probe), and then reacted with the second biomolecule structure detection probe as a secondary antibody. Specifically, immunostaining was performed as follows.

Cells were fixed by adding 4% paraformaldehyde (paraformaldehyde, Nacalai Tesque, Inc.) to the cells cultured in a cell culture dish and allowing the cells to react for 15 minutes. Permeabilization was performed by adding 0.5% Triton X-100 to the fixed cells and allowing the cells to react for 5 minutes. After that, blocking was performed by adding a blocking solution (Blocking One-P, Nacalai Tesque, Inc.) and reacting for 10 minutes. Subsequently, a primary antibody (Anti-βActin, Anti-H2AZ, and the like) was diluted to an appropriate concentration with a 10% blocking solution and allowed to react with the cells at room temperature for 45 minutes. After that, the cells were washed with PBS three times for 5 minutes, and a linker-marked rat anti-mouse IgG antibody or goat anti-rabbit IgG antibody (500-fold dilution with 10% blocking solution) was allowed to react with the cells at room temperature for 45 minutes. After the reaction, the cells were washed with PBS three times for 5 minutes, and reacted with fluorescence-marked avidin diluted 1000-fold with a 10% blocking solution at room temperature for 45 minutes. After the reaction, the cells were washed with PBS three times for 5 minutes, and fluorescence observation was performed. After the fluorescence observation, the cells were added with 50 mM TCEP and treated at room temperature for 30 minutes. After the reaction, the cells were washed with PBS three times for 5 minutes, and fluorescence observation was performed again.

### <Results>

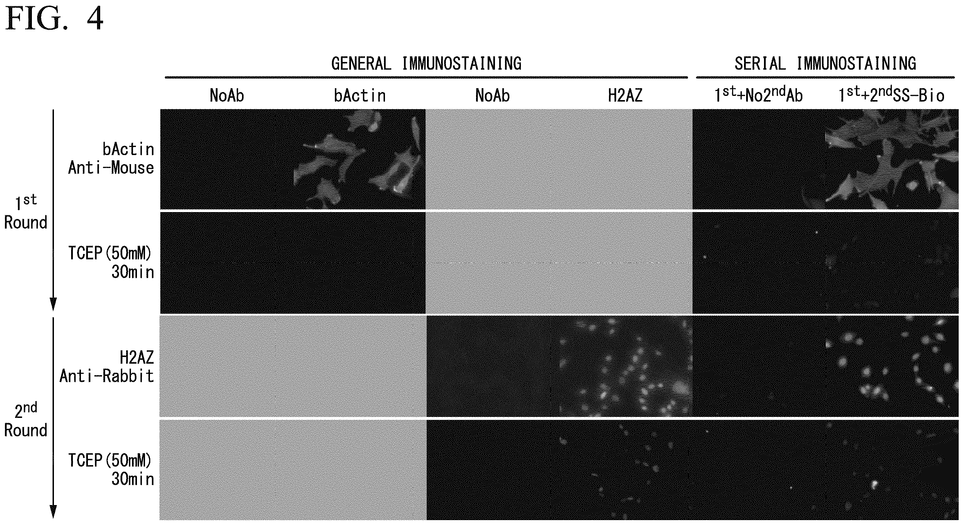

The results are shown in FIG. 4. As shown in FIG. 4, by reacting the mouse anti-β-actin antibody as a primary antibody, and then reacting the first biomolecule structure detection probe, β-actin could be detected based on FITC fluorescence (1st row image). Subsequently, by treatment with 50 mM TCEP for 30 minutes, FITC was released and FITC fluorescence disappeared (2nd row, rightmost image).

In addition, by reacting the rabbit anti-H2AZ antibody as a primary antibody, and then reacting the second biomolecule structure detection probe, H2AZ could be detected based on FITC fluorescence (3rd row image). Subsequently, by treatment with 50 mM TCEP for 30 minutes, FICT was released and the FITC fluorescence disappeared (4th row, rightmost image).

From the above results, by using a biomolecule structure detection probe in which a specific binding substance was linked to a labeling substance via a linker including a disulfide bond, it was confirmed that the labeling substance could be released at any timing. In addition, it was confirmed that immunostaining could be performed repeatedly.

### [Example 2]

As the biomolecule structure detection probe, the second biomolecule structure detection probe prepared in Example 1 was used. A rabbit anti-H2AZ antibody was used as the primary antibody and reacted with the sample in the same manner as above, and then reacted with the biomolecule structure detection probe as the secondary antibody (Staining). After that, treatment was performed with 0 mM, 1 mM, 5 mM, 20 mM, or 50 mM TCEP-HCl (TCEP). In addition, Alexa555-marked goat anti-rabbit antibody (Goat anti-Rabbit IgG, Thermo Fisher) or avidin-labeled FITC was allowed to react with the TCEP-treated sample (Re-staining).

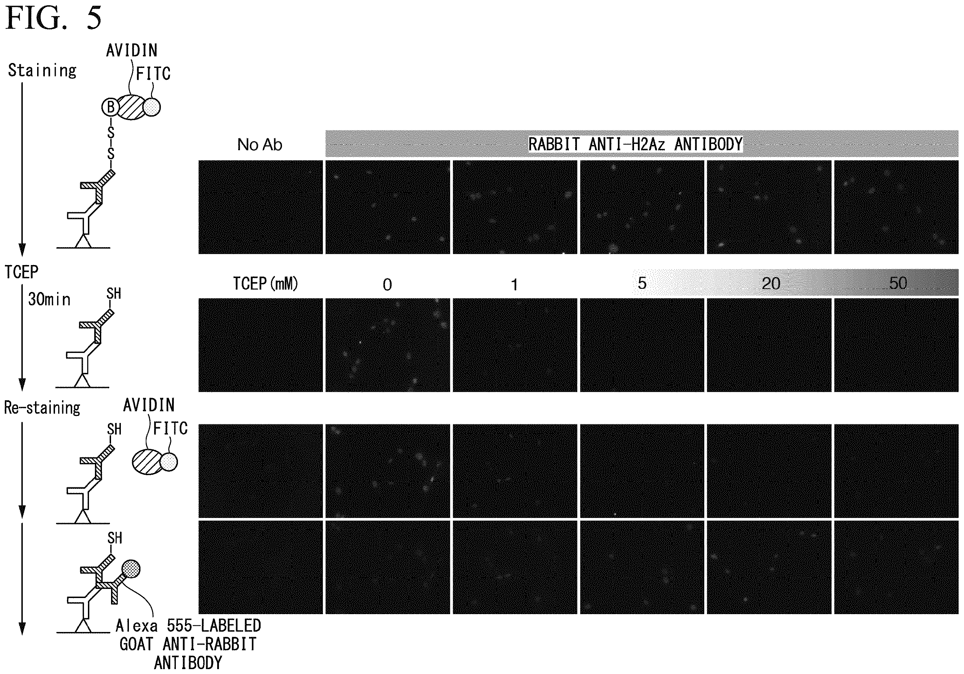

The results are shown in FIG. 5. As shown in FIG. 5, the rabbit anti-H2AZ antibody was reacted with a primary antibody, and then H2AZ could be detected based on FITC fluorescence by reacting the biomolecule structure detection probe (FIG. 5, 1st row image). Subsequently, by treatment with 0 to 50 mM TCEP for 30 minutes, FICT was released in a TCEP concentration-dependent manner, and FITC fluorescence disappeared (FIG. 5, 2nd row image). In addition, in a case where biotin-labeled FITC was allowed to react with the TCEP-treated sample, almost no FITC fluorescence was detected. From this result, it was confirmed that a disulfide bond was cleaved by TCEP and avidin was released (FIG. 5, 3rd row image). Subsequently, Alexa555-labeled goat anti-rabbit antibody was allowed to react with the TCEP-treated sample, and Alexa555 fluorescence could be detected (FIG. 5, 4th row). From this result, it was confirmed that the primary antibody remained bound to H2Az in the sample.

### [Example 3, Comparative Example 1]

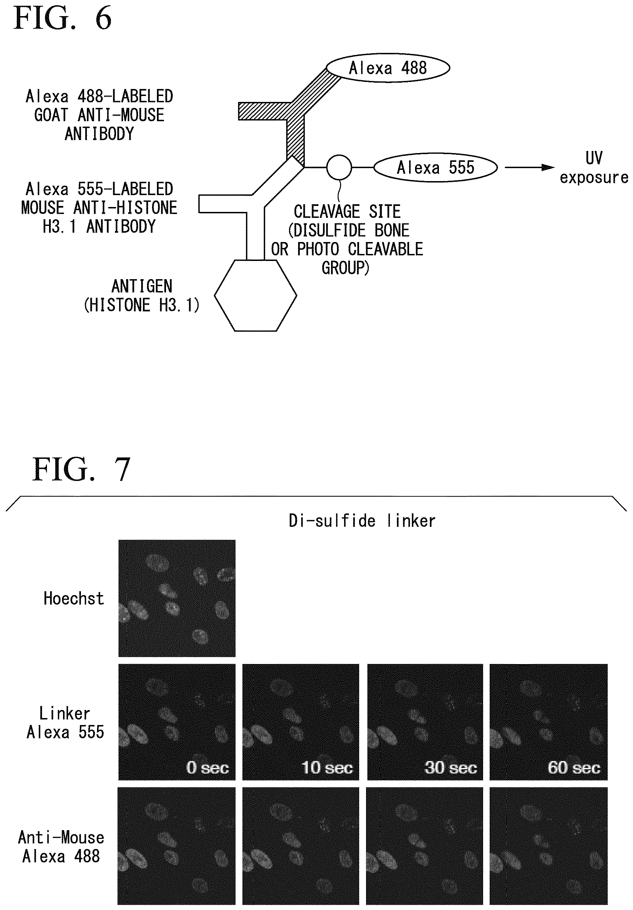

An outline of the immunostaining method of Example 3 and Comparative Example 1 is schematically shown in FIG. 6. In Example 3 and Comparative Example 1, Alexa 555-labeled mouse anti-histone H3.1 antibody was used as the primary antibody, and Al2xa 488-labeled goat anti-mouse antibody was used as the secondary antibody. The Alexa 555-labeled antibody used as the primary antibody includes a disulfide bond or a 2-nitrobenzyl group at a linker moiety between the antibody and Alexa 555.

### <Example 3>

A biomolecule structure detection probe was prepared in the same method as preparation of the first biomolecule structure detection probe, except that a mouse anti-histone H3.1 antibody (Anti-H3.1 antibody, prepared at the Ohkawa Laboratory, Medical Institute of Bioregulation, Kyushu University) was used as a specific binding substance and avidin-labeled Alexa 555 (Streptavidin, Alexa Fluor 555 conjugate, Thermo Fisher) was used as a labeling substance.

Immunostaining was performed in the same method, except that the biomolecule structure detection probe was used as the primary antibody and Alexa 488-labeled goat anti-mouse antibody (Goat anti-Mouse IgG Alexa Fluor 488, Thermo Fisher) was used as the secondary antibody. In addition, Hoechst staining was performed and imaging was performed at a wavelength of 405 nm.

The results are shown in FIG. 7. As shown in FIG. 7, Alexa 555 fluorescence could be detected even after 60 seconds from the start of irradiation of excitation wavelength.

### <Comparative Example 1>

An antibody in which Alexa 555 was bonded to a mouse anti-histone H3.1 antibody (Anti-H3.1 antibody, prepared at Ohkawa Laboratory, Medical Institute of Bioregulation, Kyushu University) via a photo cleavable linker was used as a primary antibody. As the photo cleavable linker, those including a 2-nitrobenzyl group as a photo cleavable group was used (PC-Biotin-PEG4-NHS carbonate, Funakoshi).

Immunostaining was performed in the same method as in Example 1, except that the Alexa 555-labeled mouse anti-histone H3.1 antibody including the photo cleavable linker was used as the primary antibody. In addition, Hoechst staining was performed and imaging was performed at a wavelength of 405 nm.

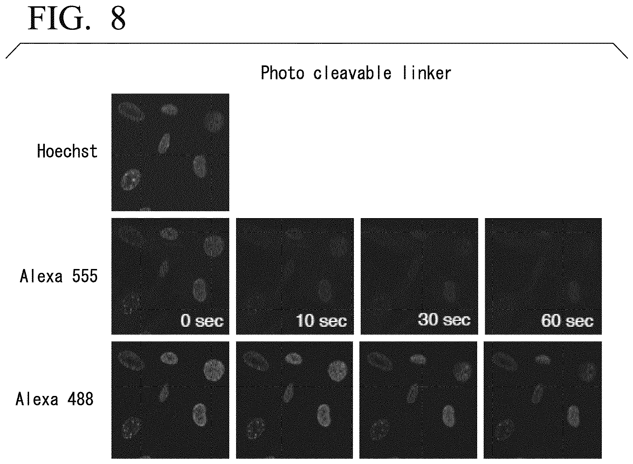

The results are shown in FIG. 8. As shown in FIG. 8, the Alexa 555 fluorescence was discolored from the start of irradiation of the excitation wavelength, and it was difficult to detect the Alexa 555 fluorescence after 30 seconds and after 60 seconds. On the other hand, since the Alexa 488 fluorescence was detected, it was confirmed that the primary antibody remained. Therefore, it was considered that the discoloration of the Alexa 555 was caused as the photo cleavable group was cleaved by irradiation with excitation light and thus the Alexa 555 was released.

From the results described above, it was confirmed that the method of performing release of the labeling substance by a disulfide bond is more excellent than the method of using a photo cleavable group.

### [Reference Example 1]

Immunostaining was performed in the same manner as that described above, using a rabbit anti-H2AZ antibody (Anti-H2AZ, Abeam) as a primary antibody and an Alexa 488-labeled goat anti-rabbit antibody as a secondary antibody (Staining). After that, treatment was performed with 0 mM, 5 mM, 20 mM, or 50 mM TCEP-HCl for 30 minutes (TCEP). Subsequently, staining was performed again with Alexa 488-labeled goat anti-rabbit antibody (Restaining).

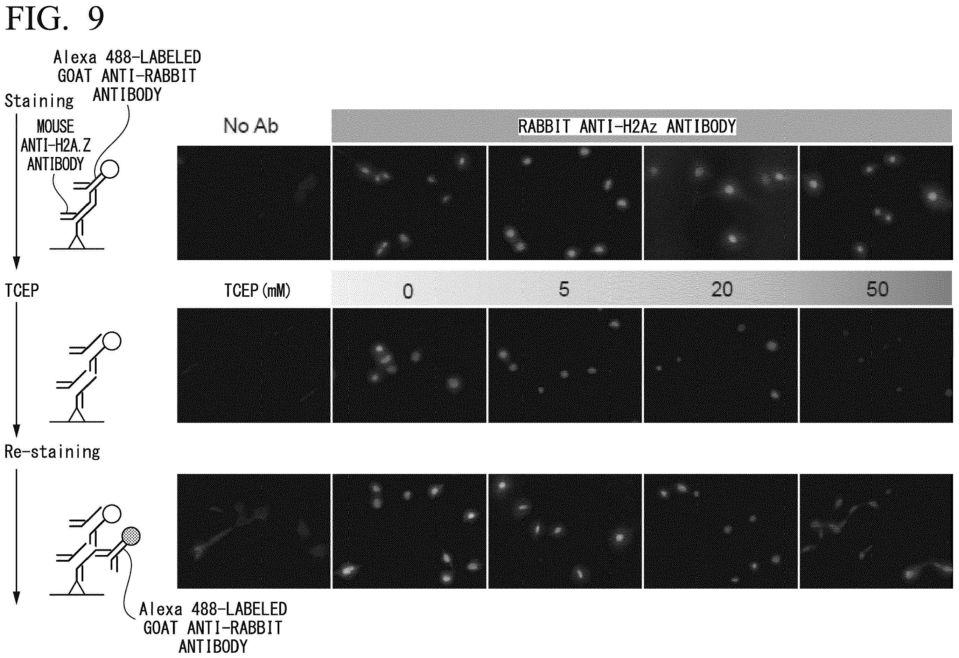

The results are shown in FIG. 9. As shown in FIG. 9, in the TCEP treatment, Alexa 488 fluorescence derived from the reacted antibody was detected (FIG. 9, 2nd row image). In addition, the TCEP-treated sample was reacted with Alexa 488-labeled goat anti-rabbit antibody, Alexa 488 fluorescence was detected, and thus it was confirmed that the primary antibody remained in the sample. Combining these results with the results of Examples 1 and 2, it was considered that the disulfide bond in the linker linking the labeling substance and the specific binding substance was cleaved by TCEP more efficiently than the disulfide bond in the antibody.

### [Example 4]

### <Preparation of biomolecule structure detection probe>

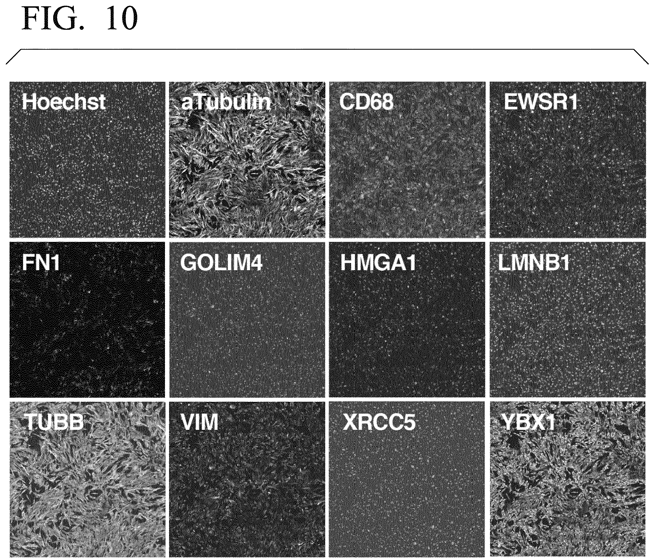

Each biomolecule structure detection probe was prepared in the same manner as in Example 1, except that an antibody specific to each protein shown in FIG. 10 was used as a specific binding substance.

### <Serial immunostaining>

Cells were fixed by adding 4% paraformaldehyde (paraformaldehyde, Nacalai Tesque, Inc.) to the cells cultured in a cell culture dish and allowing the cells to react for 15 minutes. Permeabilization was performed by adding 0.5% Triton X-100 to the fixed cells and allowing the cells to react for 5 minutes. After that, blocking was performed by adding a blocking solution (Blocking One-P, Nacalai Tesque, Inc.) and reacting for 10 minutes. Subsequently, a linker-labeled anti-aTublin mouse IgG antibody (500-fold diluted with 10% blocking solution) was allowed to react with the cells at room temperature for 30 minutes. After the reaction, the cells were washed with PBS three times for 5 minutes, and fluorescence observation was performed. After the fluorescence observation, the cells were added with 50 mM TCEP and treated at room temperature for 30 minutes.

Immunostaining was performed in the same manner as that described above, except that a linker-labeled anti-CD68 mouse IgG antibody was used for the TCEP-treated sample, and fluorescence observation was performed. After that, TCEP treatment was performed in the same manner as described above. The same treatments were repeated using a mouse IgG antibody that specifically binds to each protein shown in FIG. 10.

### <Results>

The results are shown in FIG. 10. By using a biomolecule structure detection probe including an antibody to each protein as a specific binding substance, each protein could be detected by immunostaining. It was confirmed that the TCEP treatment removed the labeling substance of the previously performed immunostaining, and there was no influence on the subsequent immunostaining.

### [Example 5]

### <Preparation of biomolecule structure detection probe>

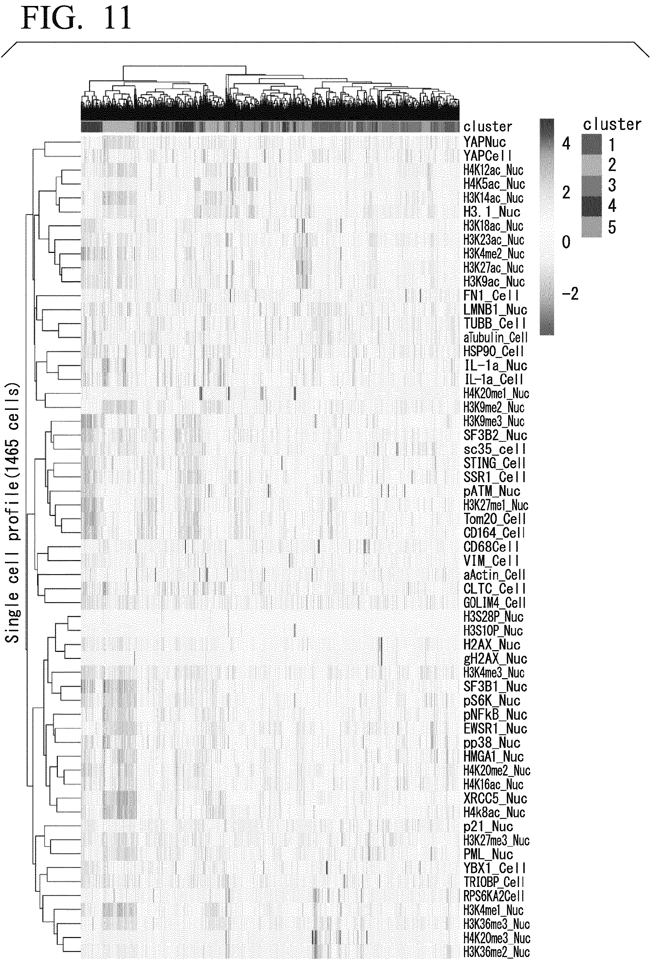

Each biomolecule structure detection probe was prepared in the same manner as in Example 1, except that an antibody specific to each protein shown in FIG. 11 was used as a specific binding substance.

### <Serial immunostaining>

Serial immunostaining was performed in the same manner as in Example 10, except that an antibody specific to each protein shown in FIG. 11 was used.

### <Quantification of serial immunostaining signals>

From immunostained images for each protein obtained by serial immunostaining, each protein signal was quantified at a single-cell level using MATLAB (registered trademark) (MathWorks, Inc.). The results are shown as proteome data in FIG. 11. Serial immunostaining showed that proteome analysis was possible at the single-cell level.

### <Grouping of cells by proteome>

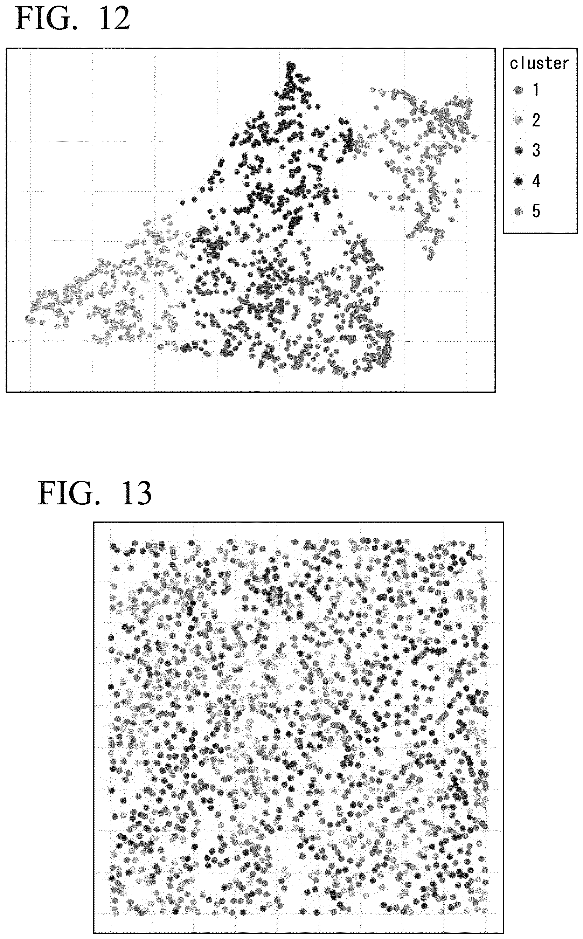

The data shown in FIG. 11 was dimensionality reduction (UMAP) and grouped into five groups by similar cell types. The results are shown in FIG. 12. Serial immunostaining showed that grouping of cells by proteome analysis was possible.

### <Distribution analysis of cell population>

Positions of cells present in the immunostained image were shown as dots, and the positions of the cells in the five groups grouped above were shown as dots with color corresponding to each group. The results are shown in FIG. 13.

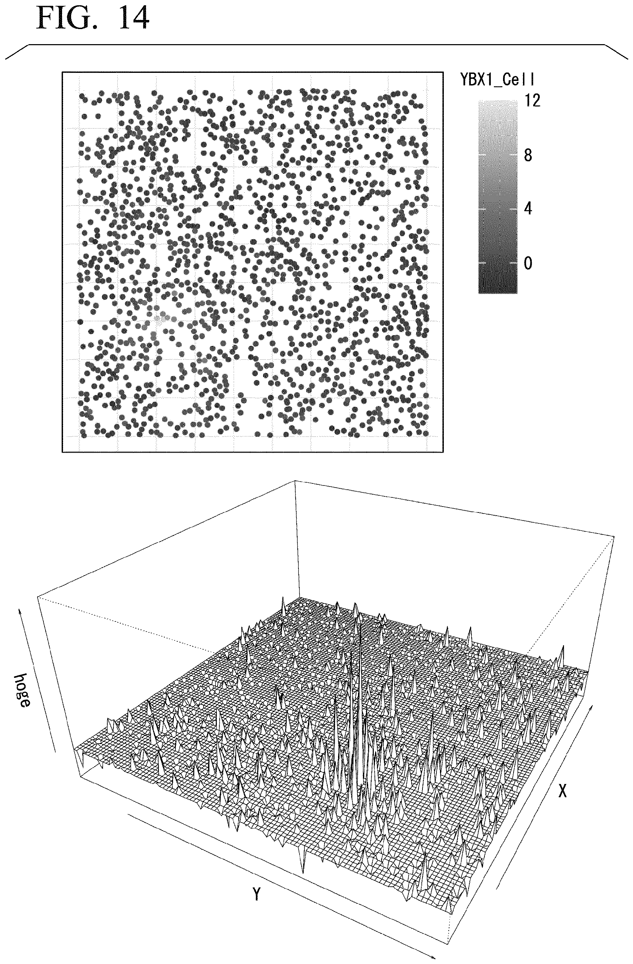

The results of superimposing a quantitative value of expression of a specific protein on the cell position information obtained in FIG. 13 are shown in FIG. 14. From the results of FIG. 14, it was shown that cells with high expression of a specific protein are spatially biased.

From the results described above, it was shown that proteome analysis is possible by performing serial staining using a biomolecule structure detection probe including a disulfide bond in the linker moiety.

### [Industrial Applicability]

According to the present invention, there is provided a biomolecule structure detection probe, a biomolecule structure detection kit, and a method for detecting a biomolecule structure, in which the same labeling substance can be repeatedly used without using a photo cleavable label.

Although the preferred embodiments of the present invention have been described and shown above, it is to be understood that they are intended to be illustrative of the present invention and should not be taken as limiting. Additions, omissions, substitutions, and other changes can be made without departing from the spirit or scope of the present invention. Accordingly, the present invention should not be viewed as limited by the above description, but only by the scope of the appended claims.

### [Reference Signs List]

1: Sample containing cells
10a, 10b: Biomolecule
20a, 20b, 21a, 21b: Specific binding substance
30a, 30b: Labeling substance
40a, 40b: Linker
P1, P2, P3, P4: Biomolecule structure detection probe

## Claims

1. A biomolecule structure detection probe,
wherein a specific binding substance having a specific binding activity to a biomolecule structure is linked to a labeling substance via a linker including a disulfide bond.

2. The biomolecule structure detection probe according to Claim 1,
wherein the specific binding substance is an antibody.

3. The biomolecule structure detection probe according to Claim 1 or 2,
wherein the labeling substance is a fluorescent dye.

4. A biomolecule structure detection kit comprising:
the biomolecule structure detection probe according to any one of Claims 1 to 3; and
a reagent for cleaving a disulfide bond.

5. A biomolecule structure detection kit comprising:
a linker for linking a specific binding substance having a specific binding activity to a biomolecule structure with a labeling substance, the linker including a disulfide bond;
a labeling substance bonded to or capable of being bonded to the linker; and
a reagent for cleaving the disulfide bond.

6. The biomolecule structure detection kit according to Claim 5,
wherein the specific binding substance is an antibody.

7. The biomolecule structure detection kit according to Claim 5 or 6,
wherein the labeling substance is a fluorescent dye.

8. A method for detecting a biomolecule structure comprising:
step (A) of detecting a first biomolecule structure in a sample containing cells, using a first biomolecule structure detection probe in which a specific binding substance having a specific binding activity to the first biomolecule structure is linked to a first labeling substance via a linker including a disulfide bond; and
step (B) of cleaving the disulfide bond in the first biomolecule structure detection probe to release the first labeling substance.

9. The method for detecting a biomolecule structure according to Claim 8, further comprising:
step (C) of detecting a second biomolecule structure in the sample, using a second biomolecule structure detection probe in which the specific binding substance having a specific binding activity to the second biomolecule structure is linked to a second labeling substance via a linker including a disulfide bond, after the step (B).

10. The method for detecting a biomolecule structure according to Claim 9,
wherein the first labeling substance and the second labeling substance are the same labeling substances.

11. The method for detecting a biomolecule structure according to any one of Claims 8 to 10,
wherein the first biomolecule structure is a biomolecule structure included in a first primary probe that is specifically bound to a third biomolecule structure included in the cell.

12. The method for detecting a biomolecule structure according to Claim 9 or 10,
wherein the first biomolecule structure is a biomolecule structure included in a first primary probe that is specifically bound to a third biomolecule structure included in the cell, and
the second biomolecule structure is a biomolecule structure included in a second primary probe that is specifically bound to a fourth biomolecule structure included in the cell.
